# EUROPÄISCHE PATENTSCHRIFT

(11) **EP 4 314 768 B1**
(45) Veröffentlichungstag und Bekanntmachung des Hinweises auf die Patenterteilung: **03.06.2026**
(21) Anmeldenummer: 22712243.9
(22) Anmeldetag: 15.03.2022
(51) Int. Cl.: G01N 21/31, G01N 21/94, G01N 21/84

(54) **BESTIMMUNG EINES MISCHUNGSVERHÄLTNISSES ZWEIER BESTANDTEILE EINES TEXTILEN FASERGEBILDES**
DETERMINATION OF A MIXING RATIO OF TWO COMPONENTS OF A TEXTILE FIBER STRUCTURE
DÉTERMINATION D'UN TAUX DE MÉLANGE DE DEUX COMPOSANTS D'UNE STRUCTURE DE FIBRES TEXTILE

(30) Priorität: 26.03.2021 CH 3232021
(43) Veröffentlichungstag der Anmeldung: 07.02.2024
(73) Patentinhaber: Uster Technologies AG, 8610 Uster (CH)
(72) Erfinder: JACOB, Rainer, 8320 Fehraltorf (CH)
(74) Vertreter: Pliska, Pavel
(86) Internationale Anmeldenummer: PCT/CH2022/000002
(87) Internationale Veröffentlichungsnummer: WO 2022/198342

(56) Entgegenhaltungen:
- WO-A1-2019/051620
- US-A1- 2017 241 839
- PRIORE RYAN J ET AL: "Spectral imaging of chemical compounds using multivariate optically enhanced filters integrated with InGaAs VGA cameras", PROCEEDINGS OF SPIE; [PROCEEDINGS OF SPIE ISSN 0277-786X VOLUME 10524], SPIE, US, vol. 9824, 12 May 2016 (2016-05-12), pages 98240P - 98240P, XP060068108, ISBN: 978-1-5106-1533-5, DOI: 10.1117/12.2222784

## Beschreibung

### FACHGEBIET

Die vorliegende Erfindung liegt auf dem Gebiet der Qualitätsüberwachung in der Textilindustrie. Sie betrifft ein Verfahren zur Bestimmung eines Mischungsverhältnisses zweier Bestandteile eines textilen Fasergebildes gemäss dem ersten Patentanspruch. Eine bevorzugte Anwendung ist die Detektion von Fremdmaterialien in einem textilen Fasergebilde wie Faserflocken, Faservlies, Faserband, Vorgarn, Garn, Gewebe, Gestrick oder Vlies.

### STAND DER TECHNIK

Fremdmaterialien im Garn stellen eines der grossen Probleme heutiger Spinnereien dar. Es handelt sich dabei um Materialien, die sich vom Grundmaterial der Garnfasern, z. B. Baumwollfasern, unterscheiden. Sie können verschiedenen Ursprungs sein, wie z. B. Rückstände der Transportverpackung (Kunststoffverpackungen, Schnüre), Zivilisationsverunreinigungen (Russteile, Plastiksäcke) oder Rückstände von Lebewesen (menschliche oder tierische Haare, Pflanzenstängel). Fremdmaterialien führen zu Fadenbrüchen beim Spinnen und Weben, nehmen Farbstoff in anderer Weise an als das Grundmaterial und beeinflussen das Aussehen des textilen Endproduktes. Sie vermindern wesentlich den Wert des Endproduktes. Eine Übersicht über Gewebefehler, die durch Fremdmaterialien verursacht sind, und Empfehlungen zu ihrer Verminderung gibt Abs. 3.8 des USTER^{®} *NEWS BULLETIN NO. 47* "The origins of fabric defects - and ways to reduce them", Uster Technologies AG, März 2010.

Fremdmaterialien können in verschiedenen Stufen des Garnherstellungsprozesses erkannt und allenfalls ausgeschieden werden.

Der Putzereiprozess ist Teil des Garnherstellungsprozesses und ist dem Kardierprozess vorgeschaltet. Ziel ist eine Aufbereitung des Rohmaterials, damit dieses in möglichst konstanter Qualität und frei von Verunreinigungen dem Kardierprozess zugeführt werden kann. Er umfasst das Öffnen des Rohmaterials, die Zuführung desselben in den Verarbeitungsprozess sowie das Mischen und Grobreinigen des zugeführten Materials. Je nach Auslegung des Prozesses können dabei einzelne Arbeitsschritte mehrmals durchlaufen werden oder auch wegfallen. Das Material hat in dieser Prozessstufe die Form von Faserflocken (zum Beispiel bei Baumwolle und Wolle) oder Schnipseln (bei Kunstfasermaterial). Der Transport des Materials erfolgt mit einem Luftstrom, der die verschiedenen Anlagen im Putzereiprozess verbindet.

Der Spinnprozess ist ein weiterer Teil des Garnherstellungsprozesses und ist dem Kardierprozess mittelbar oder unmittelbar nachgeschaltet. In diesem Prozess wird aus einem Faserband, z. B. dem Zwischenprodukt einer Karde, oder einem Vorgarn das Garn als Endprodukt gesponnen. Dabei wird das Vorgarn oder das Faserband durch Strecken und Verdrehen in seine endgültige Form, das Garn, umgewandelt. Während des Spinnens wird das Garn auf Spindeln aufgewickelt. Anschliessend werden die Spindeln auf grosse Spulen umgespult. Der Transport des Materials erfolgt in Form von Spindeln und Spulen.

Die Ausreinigung von Fremdmaterialien kann grundsätzlich in die folgenden drei Schritte unterteilt werden:
1) Erkennung des Fremdmaterials;
2) räumliche/zeitliche Lokalisation des Fremdmaterials innerhalb des Prüfguts; und
3) Ausscheidung des Fremdmaterials.

Im Putzereiprozess kann die Fremdmaterialreinigung manuell durchgeführt werden, bevor das Rohmaterial dem automatischen Verarbeitungsprozess zugeführt wird, oder die Reinigung kann maschinell durch eine entsprechende Anlage innerhalb des Putzereiprozesses durchgeführt werden. Heutzutage ist die maschinelle Reinigung üblich.

Bei der maschinellen Reinigung erfolgt die Erkennung und Lokalisation mit Hilfe von Detektionsvorrichtungen, welche Unterschiede in einem bestimmten Charakteristikum innerhalb des Materialstroms erkennen. Nicht abschliessend seien hier genannt: Reflexion und Transmission von elektromagnetischer Strahlung oder Fluoreszenz. In einfachsten Anwendungen wird mit optischen Detektionsvorrichtungen das menschliche Auge nachgeahmt und der Farbeindruck des Materialstroms analysiert, wobei entsprechende Farbunterschiede erkannt werden. Die US-6,452,157 B1 offenbart eine Vorrichtung zum Erkennen von Verunreinigungen, Fremdmaterialien und -fasern in textilem Fasermaterial. Die Vorrichtung hat mindestens zwei Lichtquellen, die das Fasermaterial abwechselnd mit unterschiedlicher Farbe beleuchten. Ausserdem ist ein Sensor vorgesehen, der die Farben des vom Fasermaterial reflektierten Lichtes empfängt.

Zur Detektion von Fremdmaterialien, die für sichtbares Licht transparent sind oder eine ähnliche Farbe wie das Rohmaterial aufweisen, braucht es jedoch raffiniertere Detektionsvorrichtungen. In diesem Fall kann der Materialstrom mit Hilfe von solcher elektromagnetischen Strahlung analysiert werden, welche vom menschlichen Auge nicht wahrgenommen wird (Ultraviolett oder Infrarot). Dabei wird die Materialzugehörigkeit anhand charakteristischer Signaturen (zum Beispiel Abfolge spezifischer Absorptionsbanden) im reflektierten oder transmittierten Spektrum der elektromagnetischen Strahlung ermittelt. Die Unterscheidung der charakteristischen Signaturen wird dabei umso genauer, je mehr Charakteristika (zum Beispiel Absorptionsbanden) innerhalb der Signatur für die Unterscheidung genutzt werden. Momentan erfordert jedes Charakteristikum innerhalb der Signatur einen dedizierten Sensor innerhalb der Detektionsvorrichtung, so dass dieser nur auf das Vorhandensein/Fehlen dieses einen Charakteristikums anspricht. Je mehr Charakteristika verwendet werden sollen, desto komplexer wird die Detektionsvorrichtung. Das Eingangssignal muss entsprechend auf die Anzahl der Sensoren aufgespaltet werden und verliert dadurch an Intensität. Weiterhin gibt es für bestimmte Charakteristika derzeit keine räumlich, sondern lediglich zeitlich auflösende Sensoren. Dies erfordert eine der Detektionsvorrichtung vorgeschaltete Einrichtung, welche Zeit und Ort miteinander verknüpft. Alternativ kann die einfallende elektromagnetische Strahlung zeitlich moduliert und an die Charakteristika angepasst werden. Dies ist jedoch für den nicht-sichtbaren Bereich des elektromagnetischen Spektrums teilweise mit grossem Aufwand verbunden.

Bei der Unterscheidung des Materials anhand von Farbunterschieden, wie es im Spinnprozess üblich ist, liegen die genannten Charakteristika im sichtbaren Spektralbereich. Da der Farbeindruck ebenfalls auf die spezifische Reflexion/Transmission bestimmter Anteile des eingestrahlten Wellenlängenspektrums zurückzuführen ist, muss auch in diesem Fall jedes Charakteristikum einzeln detektiert werden. Hierzu kann entweder eine zeitliche Farbmodulation des Eingangssignals erfolgen, oder das vom Garn veränderte Ausgangssignal muss, wie bereits erläutert, in die einzelnen Charakteristika zerlegt werden. Im ersteren Fall verschlechtert sich die Ortsauflösung, im letzteren das Signal-Rausch-Verhältnis. Je mehr Farben eingesetzt werden, umso stärker wirken sich diese Nachteile aus. Deshalb haben sich in der Praxis Verfahren etabliert, die nur eine oder maximal zwei Farben einsetzen. Ein Garnreiniger, der das Garn mit mehreren verschiedenfarbigen Lichtkomponenten abtastet, ist aus der WO-2011/026249 A1 bekannt.

Multivariate optische Filter sind eine besondere Kategorie optischer Transmissionsfilter oder Reflexionsfilter. Die Filtereigenschaften der multivariaten optischen Filter werden an eine bestimmte chemische Signatur angepasst. Verschiedene Charakteristika der Signatur können gleichzeitig und unabhängig voneinander in einem einzelnen Filter genutzt werden. Multivariate optische Elemente erlauben somit die Materialidentifikation anhand der chemischen Signatur. Entspricht das Eingangssignal des Filters exakt der abgestimmten Signatur, so passiert das Signal das multivariate optische Filter ungehindert. Weicht das Eingangssignal von der Signatur ab, so wird es beim Passieren des Filters gedämpft. Je stärker die Abweichung ist, desto stärker die Dämpfung. Multivariate optische Filter ermöglichen neben der Materialunterscheidung auch die Bestimmung eines Mischungsverhältnisses auf Basis der durch die Mischung veränderten chemischen Signatur. Das spezifische Transmissions- oder Reflexionsverhalten multivariater optischer Filter wird mit Hilfe der Methode der partiellen kleinsten Quadrate aus den chemischen Signaturen der zu unterscheidenden Materialien gewonnen.

Die US-2017/0241839 A1 offenbart ein optisches Rechengerät zur Analyse einer Probe. Das optische Rechengerät beinhaltet eine Lichtquelle zur Beleuchtung der Probe, mehrere identische integrierte Rechenelemente in Form von dielektrischen Interferenzfiltern und einen Detektor. Das von der Lichtquelle ausgesandte Licht interagiert mit der Probe und den integrierten Rechenelementen und wird vom Detektor empfangen. Die Erkennungsempfindlichkeit optischer Rechengeräte kann durch die Kombination mehrerer integrierter Rechenelemente verbessert werden.

Der Artikel «Spectral imaging of chemical compounds using multivariate optically enhanced filters integrated with InGaAs VGA cameras" von R. J. Priore und N. Jacksen, Proceedings of SPIE, Bd. 9824, S. 98240P-1 bis 98240P-10, 12. Mai 2016, stellt Algorithmen und multivariate optische Filter zur Identifikation von chemischen Stoffen mit Hilfe von leistungsstarken InGaAs-VGA-Detektoren vor.

Aus der WO-2019/051620 A1 sind ein Messinstrument und ein entsprechendes Verfahren zum Ermitteln einer Fasermischungszusammensetzung und/oder eines Fasermischungsverhältnisses in einem Eingangsmaterial bekannt. Elektromagnetische Strahlungsquellen richten Strahlung auf das Eingangsmaterial an ersten und zweiten Stellen, und Strahlungssensoren sind zum Empfangen der transmittierten oder reflektierten Strahlung eingerichtet. Ein Steuergerät verarbeitet die Signale der Sensoren, um die Fasermischungszusammensetzung und/oder das Fasermischungsverhältnis im Eingangsmaterial zu bestimmen.

### DARSTELLUNG DER ERFINDUNG

Es ist die Aufgabe der vorliegenden Erfindung, ein Verfahren zur Bestimmung eines Mischungsverhältnisses zweier Bestandteile eines textilen Fasergebildes anzugeben, welches die obigen Nachteile vermeidet. Das Verfahren soll eine hohe Ortsauflösung und die Verwendung bildgebender, räumlich auflösender Strahlungssensoren ermöglichen. Gleichzeitig soll das Signal-Rausch-Verhältnis hoch sein.

Diese und andere Aufgaben werden durch das erfindungsgemässe Verfahren gelöst, wie es im ersten Patentanspruch definiert ist. Vorteilhafte Ausführungsformen sind in den abhängigen Patentansprüchen angegeben.

Die Erfindung beruht auf der Idee, ein Spektralfilter spezifisch in Bezug auf die Bestandteile des textilen Fasergebildes auszulegen, so dass eine vom Strahlungssensor empfangene Strahlungsintensität eine monotone Funktion des Mischungsverhältnisses der beiden Bestandteile ist. Ausserdem soll der Transmissionsgrad oder der Reflexionsgrad im betrachteten Spektralband mindestens ein lokales Maximum und mindestens ein lokales Minimum aufweisen, so dass mehrere charakteristische Wellenlängen berücksichtigt werden. Weiterhin umfasst die Erfindung, dass das genannte Spektralfilter auch auf das Verhältnis verschiedener Charakteristika, z. B. Farbeindrücke, ausgelegt werden kann und so auch Farbunterschiede artgleicher Materialien ohne Signalverlust detektierbar macht.

Die Auslegung des Spektralfilters erfolgt dabei anhand der spezifischen chemischen und/oder farblichen Signaturen der beiden Bestandteile. Als Beispiel können einzelne Charakteristika durch eine hohe Transmission verstärkt werden, während andere Charakteristika durch niedrige Transmission gedämpft werden. Die Kombination von Verstärkung und Dämpfung in dem Spektralfilter ermöglicht die Bestimmung des Mischungsverhältnisses der beiden Bestandteile anhand des aus dem Spektralfilter austretenden Signals. Als Beispiel würden Materialien und/oder Farben ein hohes Ausgangssignal erzeugen, für die das Spektralfilter optimiert wurde, wohingegen nicht optimierte Materialien und/oder Farben ein tiefes Ausgangssignal verursachen.

Das erfindungsgemässe Verfahren dient zur Bestimmung eines Mischungsverhältnisses zweier Bestandteile eines textilen Fasergebildes. Elektromagnetische Strahlung in einem Spektralband wird von einer Strahlungsquelle in Richtung des textilen Fasergebildes gesendet. Mindestens ein Teil der elektromagnetischen Strahlung wechselwirkt mit dem textilen Fasergebilde. Mindestens ein Teil der elektromagnetischen Strahlung wird nach der Wechselwirkung mit dem textilen Fasergebilde von einem Strahlungssensor empfangen. Mindestens ein Teil der elektromagnetischen Strahlung wird vor oder nach der Wechselwirkung mit dem textilen Fasergebilde von einem Spektralfilter mit spektralen Eigenschaften in dem Spektralband gefiltert. Das Spektralfilter wird derart gewählt, dass sein Transmissionsgrad oder sein Reflexionsgrad in dem Spektralband mindestens ein lokales Maximum und mindestens ein lokales Minimum aufweist und seine spektralen Eigenschaften in dem Spektralband derart auf die spektralen Eigenschaften der Strahlungsquelle und jedes der beiden Bestandteile im textilen Fasergebilde abgestimmt sind, dass eine vom Strahlungssensor empfangene Strahlungsintensität eine monotone Funktion des Mischungsverhältnisses der beiden Bestandteile ist.

In einer Ausführungsform ist einer der zwei Bestandteile ein Grundmaterial, aus dem ein überwiegender Teil des textilen Fasergebildes besteht, und der andere der zwei Bestandteile ist ein Fremdmaterial, dessen Anteil im textilen Fasergebilde bestimmt wird.

Beim Verfahren kann das Spektralband z. B. im Wellenlängenbereich zwischen 300 nm und 2200 nm und vorzugsweise im Wellenlängenbereich zwischen 700 nm und 1900 nm liegen.

Beim Verfahren kann das Spektralband z. B. eine Breite zwischen 200 nm und 500 nm haben.

Die Strahlungsquelle kann ein einziges Strahlungselement, z. B. eine Halogenlampe, beinhalten. Alternativ kann sie mehrere Strahlungselemente beinhalten, z. B. eine Halogenlampe und eine Quecksilberdampflampe.

In einer Ausführungsform liegt das mindestens eine lokale Maximum bei derjenigen Wellenlänge bzw. denjenigen Wellenlängen der elektromagnetischen Strahlung, bei welcher bzw. bei welchen der Absolutwert der Differenz der Absorptionsgrade, der Transmissionsgrade oder der Reflexionsgrade der beiden Bestandteile ein lokales Maximum aufweist.

In einer Ausführungsform weist der Transmissionsgrad oder der Reflexionsgrad des Spektralfilters in dem Spektralband mindestens je zwei lokale Maxima und lokale Minima auf.

Das Spektralfilter kann als Reflexionsfilter oder als Transmissionsfilter ausgebildet sein.

In einer Ausführungsform ist das Spektralfilter als Interferenzfilter ausgebildet.

In einer Ausführungsform ist das Spektralfilter in den Strahlungssensor integriert.

In einer Ausführungsform ist der Strahlungssensor ortsauflösend und/oder zeitauflösend. Er kann z. B. entweder als digitale Kamera mit einem zweidimensionalen Bildwandler oder als eindimensionaler Zeilensensor ausgebildet sein.

Die in dieser Schrift verwendeten Ausdrücke «lokales Maximum», «lokales Minimum» und «monotone Funktion» werden im Sinne ihrer jeweiligen mathematischen Bedeutungen verwendet. Sie sind dem Fachmann bekannt, und ihre Definitionen können Lehr- oder Nachschlagewerken zur Mathematik entnommen werden.

Das erfindungsgemässe Verfahren erlaubt eine einfache und dennoch zuverlässige Bestimmung eines Mischungsverhältnisses zweier Bestandteile eines textilen Fasergebildes. Es vermeidet eine Aufspaltung der eintreffenden, vom textilen Fasergebilde reflektieren oder transmittierten, elektromagnetischen Strahlung auf mehrere Strahlungssensoren. Die Erfindung kommt ohne eine zeitliche Modulation des Eingangssignals aus, wodurch eine hohe Ortsauflösung erreicht wird.

### AUFZÄHLUNG DER ZEICHNUNGEN

Nachfolgend wird eine Ausführungsform der Erfindung anhand der Zeichnungen detailliert erläutert. Zur anschaulicheren Darstellung wird dabei eine Anwendung beschrieben, in der ein Anteil eines Fremdmaterials in einem Grundmaterial eines textilen Fasergebildes ermittelt wird. Dies soll jedoch die Allgemeinheit der Erfindung nicht einschränken, die sich auf die Bestimmung eines Mischungsverhältnisses zweier Bestandteile eines textilen Fasergebildes bezieht.
- Figur 1: zeigt schematisch eine Ausführungsform einer Vorrichtung zur Ausführung des erfindungsgemässen Verfahrens.
- Figur 2: zeigt verschiedene Spektren in einem gemeinsamen Spektralband, nämlich: (a) relative Intensitätsverteilung einer Halogenlampe; (b) Absorptionsgrad von Baumwolle; (c) Absorptionsgrad von Polyethylen; und (d) Transmissionsgrad eines Spektralfilters.

### AUSFÜHRUNG DER ERFINDUNG

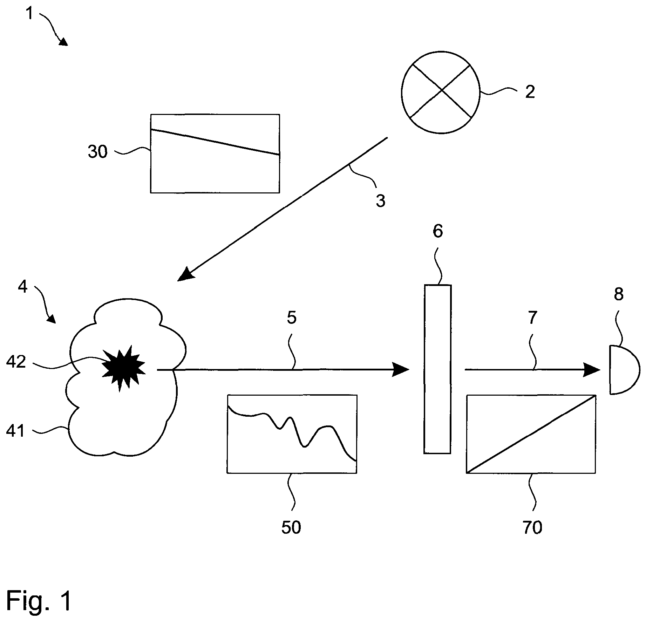

Eine Ausführungsform einer Vorrichtung 1 zur Ausführung des erfindungsgemässen Verfahrens ist schematisch in **Figur 1** dargestellt. Sie beinhaltet mindestens eine breitbandige Strahlungsquelle 2 zur Erzeugung elektromagnetischer Strahlung 3. Die erzeugte elektromagnetische Strahlung 3 hat eine für die Strahlungsquelle 2 charakteristische spektrale Intensitätsverteilung 30. In Figur 1 ist die Intensitätsverteilung 30 als schematisches Diagramm dargestellt, in welchem die Intensität als Funktion der Wellenlänge aufgetragen ist.

Mindestens ein Teil der von der Strahlungsquelle 2 erzeugten elektromagnetischen Strahlung 3 trifft auf ein zu untersuchendes textiles Fasergebilde 4 auf. Das textile Fasergebilde 4 kann z. B. eine oder mehrere Faserflocken, ein Faservlies, ein Faserband, ein Vorgarn, ein Garn, ein Gewebe, ein Gestrick oder ein Vlies sein. Im Beispiel von Figur 1 ist ohne Einschränkung der Allgemeinheit als textiles Fasergebilde 4 schematisch eine Faserflocke eingezeichnet.

Das textile Fasergebilde 4 beinhaltet zwei verschiedene Bestandteile 41, 42. Ohne Einschränkung der Allgemeinheit wird hier zur Illustration angenommen, das textile Fasergebilde 4 bestehe aus einem Grundmaterial 41, z. B. Baumwolle, und könne unter Umständen ein oder mehrere Fremdmaterialien 42 beinhalten, die sich vom Grundmaterial 41 unterscheiden. Beim Auftreffen der elektromagnetischen Strahlung 3 auf das textile Fasergebilde 4 kommt es zu einer Wechselwirkung der elektromagnetischen Strahlung 3 mit dem Grundmaterial 41 und, falls vorhanden, dem Fremdmaterial 42. Durch die Wechselwirkung wird die Intensitätsverteilung 30 der elektromagnetischen Strahlung 3 gemäss der chemischen bzw. farblichen Charakteristik der Materialien verändert. Am textilen Fasergebilde 4 reflektierte oder transmittierte Strahlung 5 hat somit eine spektrale Intensitätsverteilung 50, die sich von der Intensitätsverteilung 30 der auf das textile Fasergebilde 4 auftreffenden Strahlung 3 unterscheidet. Die Intensitätsverteilung 50 ist in Figur 1 wiederum als schematisches Diagramm dargestellt, in welchem die Intensität als Funktion der Wellenlänge aufgetragen ist.

Nach der Wechselwirkung mit dem textilen Fasergebilde 4 wechselwirkt die elektromagnetische Strahlung 5 im Ausführungsbeispiel von Figur 1 mit einem Spektralfilter 6. Die Wechselwirkung kann mittels Transmission oder Reflexion am Spektralfilter 6 erfolgen. Die spektralen Eigenschaften des Spektralfilters 6 sind speziell an einen Typ oder eine Klasse von Fremdmaterialien 42 abgestimmt. Das Spektralfilter 6 kann z. B. als Interferenzfilter ausgebildet sein. Es verändert die Intensitätsverteilung 50 der mit ihm wechselwirkenden elektromagnetischen Strahlung 5 so, dass Unterschiede zwischen dem Grundmaterial 41 und dem Fremdmaterial 42 verstärkt werden.

Entspricht die spektrale Intensitätsverteilung 50 vor dem Spektralfilter 6 jener des Grundmaterials 41, so soll die Intensität einer Strahlung 7 nach dem Spektralfilter 6 z. B. minimal sein. Entspricht die spektrale Intensitätsverteilung 50 vor dem Spektralfilter 6 dagegen jener des Fremdmaterials 42, so soll die Intensität der Strahlung 7 nach dem Spektralfilter 6 z. B. maximal sein. Besitzt die spektrale Intensitätsverteilung 50 vor dem Spektralfilter 6 Charakteristika beider Materialien 41 und 42, so soll die die Intensität der Strahlung 7 nach dem Spektralfilter 6 eine monotone Funktion des Mischungsverhältnisses der Materialien 41 und 42 entsprechen. Dies ist in Figur 1 schematisch durch ein Diagramm 70 dargestellt, das eine Intensität der Strahlung 7 nach Wechselwirkung mit dem Spektralfilter 6 als Funktion des Anteils an Fremdmaterialien 42 im textilen Fasergebilde 4 zeigt.

Das Spektralfilter 6 wandelt somit die auftreffende wellenlängenabhängige Intensitätsverteilung 50 in eine Intensitätsverteilung 70 um, die eine monotone Funktion des Mischungsverhältnisses der beiden Bestandteile 41 und 42 ist. Die Intensität der nach dem Spektralfilter 6 vorliegenden elektromagnetischen Strahlung 7 ist also ein Mass für das Mischungsverhältnis. Im hier diskutierten Beispiel ist sie ein Mass für die Präsenz und Menge des Fremdmaterials 42 im textilen Fasergebilde 4 und/oder für den Grad der Farbabweichung zwischen dem Grundmaterial 41 und dem Fremdmaterial 42.

Nach der Wechselwirkung mit dem Spektralfilter 6 wird elektromagnetische Strahlung 7 von einem breitbandigen Strahlungssensor 8 detektiert. In einer bevorzugten Ausführungsform ist der Strahlungssensor 8 ortsauflösend, und das textile Fasergebilde 4 wird mittels einer (nicht eingezeichneten) Optik auf den Strahlungssensor 8 abgebildet.

Dadurch erhält man auch Information über Anzahl, Grösse und Form der im textilen Fasergebilde 4 vorhandenen Fremdmaterialien 5. Der Strahlungssensor 8 ist vorzugsweise zeitauflösend. Er kann z. B. als digitale Kamera ausgebildet sein.

In einem vom Strahlungssensor 8 aufgenommenen Bild des textilen Fasergebildes 4 erscheinen im vorliegenden Beispiel Fremdmaterialien 42 hell vor einem dunklen Hintergrund.

In einer alternativen Ausführungsform können die spektralen Eigenschaften des Spektralfilters 6 derart auf die Strahlungsquelle 2, das Grundmaterial 41 und/oder das Fremdmaterial 42 abgestimmt sein, dass die Intensität der Strahlung 7 nach dem Spektralfilter 6 maximal ist, wenn das textile Fasergebilde 4 nur aus dem Grundmaterial 41 besteht, und mit zunehmendem Anteil an Fremdmaterial 42 abnimmt. In diesem Fall erscheinen Fremdmaterialien 42 dunkel vor einem hellen Hintergrund.

In einer anderen Ausführungsform kann das Spektralfilter 6 im Strahlengang zwischen der Strahlungsquelle 2 und dem textilen Fasergebilde 4 eingesetzt sein. Diesfalls wechselwirkt die von der Lichtquelle 2 erzeugte elektromagnetische Strahlung 3 zuerst mit dem Spektralfilter 6 und trifft danach auf das textile Fasergebilde 4 auf. Die Wirkung ist analog und ein vom Strahlungssensor 8 aufgenommenen Bild des textilen Fasergebildes 4 entspricht im Wesentlichen den gemäss den oben beschriebenen Ausführungsformen aufgenommenen Bildern.

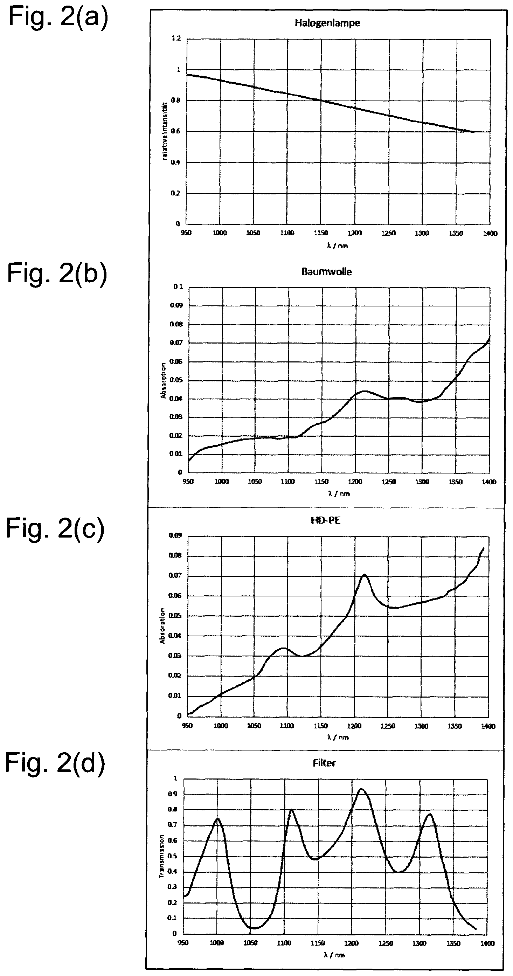

In **Figur 2(a)** ist beispielhaft die relative Intensität der von einer Halogenlampe 2 erzeugten elektromagnetischen Strahlung 3 als Funktion der Strahlungswellenlänge λ aufgetragen. Im dargestellten Spektralband (950 nm ≤ λ ≤ 1400 nm, nahes und kurzwelliges Infrarot) nimmt die relative Intensität monoton mit der Strahlungswellenlänge λ ab. Bei anderen Lichtquellen 2 kann das Intensitätsspektrum anders aussehen.

**Figuren 2(b) und 2(c)** zeigen Absorptionsspektren von Baumwolle, die ein typisches textiles Grundmaterial 41 darstellt, bzw. Polyethylen, das ein Fremdmaterial 42 sein kann. Der jeweilige Absorptionsgrad ist wiederum als Funktion der Strahlungswellenlänge λ im selben Spektralband wie in Figur 2(a) aufgetragen.

Die spektralen Eigenschaften des Spektralfilters 6 werden aus der spektralen Intensitätsverteilung 30 der Strahlungsquelle 2 sowie aus spektralen Eigenschaften - Absorptionsgrad, Reflexionsgrad und/oder Transmissionsgrad - des Grundmaterials 41 und des zu detektierenden Fremdmaterials 42 durch mehrdimensionale Variationsrechnung ermittelt. Der aus der mehrdimensionalen Variationsrechnung resultierende Regressionsvektor enthält für jede Wellenlänge im betrachteten Spektralband eine Gewichtung. Die Gewichtungen entsprechen dem Transmissions- bzw. Reflexionsgrad des Spektralfilters 6 für die betreffenden Wellenlängen. Somit ist das Spektralfilter 6 für die Detektion eines bestimmten Fremdmaterials 42 in einem bestimmten Grundmaterial 41 bei Beleuchtung mit einer bestimmten Strahlungsquelle 2 optimiert. Solche Verfahren für den Entwurf eines Spektralfilters sind an sich bekannt; ein Beispiel findet sich im Artikel «PLS-regression: a basic tool of chemometrics» von S. Wold, M. Sjöström und L.

Eriksson, Chemometrics and Intelligent Laboratory Systems, Band 58, Heft 2, 28. Oktober 2001, Seiten 109-130.

In **Figur 2(d)** ist ein beispielhafter Transmissionsgrad eines Spektralfilters 6 als Funktion der Strahlungswellenlänge λ im selben Spektralband wie in den Figuren 2(a)-2(c) aufgetragen. Im dargestellten Beispiel hat das Spektralfilter 6 im betrachteten Spektralband (950 nm ≤ λ ≤ 1400 nm) vier lokale Maxima (bei Wellenlängen von ca. λ ≈ 1000 nm, 1110 nm, 1213 nm und 1317 nm) und drei lokale Minima (bei Wellenlängen von ca. λ ≈ 1055 nm, 1145 nm und 1268 nm). Das Spektralfilter 6 verstärkt die die Unterschiede in der Absorption von Baumwolle (Figur 2(b)) und Polyethylen (Figur 2(c)), was insbesondere bei den Wellenlängen von ca. λ ≈ 1100 nm, 1210 nm und 1320 nm an den betreffenden Spektren erkennbar ist.

Durch die Optimierung der Transmission bzw. Reflexion des Spektralfilters 6 für das Fremdmaterial 42 können diejenigen Anteile der auf das Spektralfilter 6 einfallenden elektromagnetischen Strahlung 5, die aus der Wechselwirkung der Strahlung 5 mit dem Fremdmaterial 42 resultieren, das Spektralfilter 6 ungedämpft passieren. Anteile, die vom Grundmaterial 41 resultieren, werden vom Spektralfilter 6 gedämpft. Das Signal auf dem Strahlungssensor 8 ist somit hoch für das Fremdmaterial 42 und niedrig für das Grundmaterial 41. Ist der Strahlungssensor 8 als Bildsensor ausgebildet, so erscheinen auf dem vom Strahlungssensor 8 generierten Bild das Fremdmaterial 42 als helle Bildbereiche und das Grundmaterial 41 als dunkle Bildbereiche.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der durch die folgenden Ansprüche definierten Erfindung gehören.

### BEZUGSZEICHENLISTE

- 1: Vorrichtung

- 2: Strahlungsquelle

- 3: von der Strahlungsquelle erzeugte elektromagnetische Strahlung
- 30: spektrale Intensitätsverteilung

- 4: textiles Fasergebilde
- 41: Grundmaterial des textilen Fasergebildes
- 42: Fremdmaterial im textilen Fasergebilde

- 5: am textilen Fasergebilde reflektierte oder transmittierte Strahlung
- 50: spektrale Intensitätsverteilung

- 6: Spektralfilter

- 7: Strahlung nach dem Spektralfilter
- 70: Intensität der detektierten Strahlung als Funktion des Anteils an Fremdmaterialien

- 8: Strahlungssensor

## Patentansprüche

1. Verfahren (1) zur Bestimmung eines Mischungsverhältnisses zweier Bestandteile (41, 42) eines textilen Fasergebildes (4), wobei
elektromagnetische Strahlung (3) in einem Spektralband von einer Strahlungsquelle (2) in Richtung des textilen Fasergebildes (4) gesendet wird,
mindestens ein Teil der elektromagnetischen Strahlung (3) mit dem textilen Fasergebilde (4) wechselwirkt,
mindestens ein Teil der elektromagnetischen Strahlung (7) nach der Wechselwirkung mit dem textilen Fasergebilde (4) von einem Strahlungssensor (8) empfangen wird und
mindestens ein Teil der elektromagnetischen Strahlung (5) vor oder nach der Wechselwirkung mit dem textilen Fasergebilde (4) von einem Spektralfilter (6) mit spektralen Eigenschaften in dem Spektralband gefiltert wird,
**dadurch gekennzeichnet, dass**
das Spektralfilter (6) derart gewählt wird, dass
sein Transmissionsgrad oder sein Reflexionsgrad in dem Spektralband mindestens ein lokales Maximum und mindestens ein lokales Minimum aufweist und
seine spektralen Eigenschaften in dem Spektralband derart auf die spektralen Eigenschaften der Strahlungsquelle (2) und jedes der beiden Bestandteile (41, 42) im textilen Fasergebilde (4) abgestimmt sind, dass eine vom Strahlungssensor (8) empfangene Strahlungsintensität eine monotone Funktion des Mischungsverhältnisses der beiden Bestandteile (41, 42) ist.

2. Verfahren nach Anspruch 1, wobei das Spektralband im Wellenlängenbereich zwischen 300 nm und 2200 nm und vorzugsweise im Wellenlängenbereich zwischen 700 nm und 1900 nm liegt.

3. Verfahren nach Anspruch 1 oder 2, wobei das Spektralband eine Breite zwischen 200 nm und 500 nm hat.

4. Verfahren nach einem der Ansprüche 1-3, wobei einer (41) der zwei Bestandteile (41, 42) ein Grundmaterial ist, aus dem ein überwiegender Teil des textilen Fasergebildes (4) besteht, und der andere (42) der zwei Bestandteile (41, 42) ein Fremdmaterial ist, dessen Anteil im textilen Fasergebilde (4) bestimmt wird.

5. Verfahren nach Anspruch 4, wobei das Grundmaterial (41) Baumwolle und das Fremdmaterial (42) Polyethylen ist.

6. Verfahren nach einem der vorangehenden Ansprüche, wobei das mindestens eine lokale Maximum bei derjenigen Wellenlänge bzw. denjenigen Wellenlängen der elektromagnetischen Strahlung (3) liegt, bei welcher bzw. bei welchen der Absolutwert der Differenz der Absorptionsgrade, der Transmissionsgrade oder der Reflexionsgrade der beiden Bestandteile (41, 42) ein lokales Maximum aufweist.

7. Verfahren nach einem der vorangehenden Ansprüche, wobei der Transmissionsgrad oder der Reflexionsgrad des Spektralfilters (6) in dem Spektralband mindestens je zwei lokale Maxima und lokale Minima aufweist.

8. Verfahren nach einem der vorangehenden Ansprüche, wobei das Spektralfilter (6) als Reflexionsfilter oder als Transmissionsfilter ausgebildet ist.

9. Verfahren nach einem der vorangehenden Ansprüche, wobei das Spektralfilter (6) als Interferenzfilter ausgebildet ist.

10. Verfahren nach einem der vorangehenden Ansprüche, wobei das Spektralfilter (6) in den Strahlungssensor (8) integriert ist.

11. Verfahren nach einem der vorangehenden Ansprüche, wobei der Strahlungssensor (8) ortsauflösend und/oder zeitauflösend ist.

12. Verfahren nach Anspruch 11, wobei der Strahlungssensor (8) entweder als digitale Kamera mit einem zweidimensionalen Bildwandler oder als eindimensionaler Zeilensensor ausgebildet ist.

## Claims

1. Method (1) for detecting a mixture ratio of two components (41, 42) of a textile fiber structure (4), wherein
electromagnetic radiation (3) in a spectral band is transmitted from a radiation source (2) in the direction of the textile fiber structure (4),
at least a part of the electromagnetic radiation (3) interacts with the textile fiber structure (4),
at least a part of the electromagnetic radiation (7) is received by a radiation sensor (8) after interacting with the textile fiber structure (4), and
at least a part of the electromagnetic radiation (5) is filtered by a spectral filter (6) with spectral properties in the spectral band before or after interacting with the textile fiber structure (4),
**characterized in that**
the spectral filter (6) is selected such that
its transmittance or its reflectance in the spectral band has at least one local maximum and at least one local minimum, and
its spectral properties in the spectral band are adapted to the spectral properties of the radiation source (2) and each of the two components (41, 42) in the textile fiber structure (4) such that a radiation intensity received by the radiation sensor (8) is a monotonous function of the mixture ratio of the two components (41, 42).

2. Method according to claim 1, wherein the spectral band is in the wavelength range between 300 nm and 2200 nm, and preferably in the wavelength range between 700 nm and 1900 nm.

3. Method according to claim 1 or 2, wherein the spectral band has a width between 200 nm and 500 nm.

4. Method according to one of claims 1-3, wherein one (41) of the two components (41, 42) is a base material of which a predominant part of the textile fiber structure (4) consists, and the other (42) of the two components (41, 42) is a foreign material whose proportion in the textile fiber structure (4) is determined.

5. Method according to claim 4, wherein the base material (41) is cotton and the foreign material (42) is polyethylene.

6. Method according to one of the preceding claims, wherein the at least one local maximum lies at the wavelength or wavelengths of the electromagnetic radiation (3) at which the absolute value of the difference of the absorptance, the transmittance, or the reflectance of the two components (41, 42) has a local maximum.

7. Method according to one of the preceding claims, wherein the transmittance or the reflectance of the spectral filter (6) in the spectral band has at least two local maxima and local minima each.

8. Method according to one of the preceding claims, wherein the spectral filter (6) is designed as a reflection filter or as a transmission filter.

9. Method according to one of the preceding claims, wherein the spectral filter (6) is designed as an interference filter.

10. Method according to one of the preceding claims, wherein the spectral filter (6) is integrated into the radiation sensor (8).

11. Method according to one of the preceding claims, wherein the radiation sensor (8) is spatially resolving and/or time resolving.

12. Method according to claim 11, wherein the radiation sensor (8) is formed either as a digital camera with a two-dimensional image converter or as a one-dimensional line sensor.

## Revendications

1. Procédé (1) pour la détermination d'un rapport de mélange de deux composantes (41, 42) d'une structure de fibres textile (4), dans lequel
un rayonnement électromagnétique (3) dans une bande du spectre est émis par une source de rayonnement (2) en direction de la structure de fibres textile (4),
au moins une partie du rayonnement électromagnétique (3) interagit avec la structure de fibres textile (4),
au moins une partie du rayonnement électromagnétique (7) est reçue par un détecteur de rayonnement (8) après l'interaction avec la structure de fibres textile (4) et
au moins une partie du rayonnement électromagnétique (5) est filtrée avant ou après l'interaction avec la structure de fibres textile (4) par un filtre spectral (6) ayant des propriétés spectrales dans la bande du spectre,
**caractérisé en ce que**
le filtre spectral (6) est choisi de telle façon que
son coefficient de transmission ou son coefficient de réflexion dans la bande du spectre comporte au moins un maximum local et au moins un minimum local et ses propriétés spectrales dans la bande du spectre soient adaptées aux propriétés spectrales de la source de rayonnement (2) et de chacune des deux composantes (41, 42) de la structure de fibres textile (4) de telle manière qu'une intensité de rayonnement reçue par le détecteur de rayonnement (8) soit une fonction monotone du rapport de mélange des deux composantes (41, 42).

2. Procédé selon la revendication 1, dans lequel la bande du spectre se situe dans la plage de longueur d'ondes de 300 nm à 2200 nm et de préférence dans la plage de longueur d'ondes de 700 nm à 1900 nm.

3. Procédé selon la revendication 1 ou 2, dans lequel la bande du spectre a une largeur de 200 nm à 500 nm.

4. Procédé selon l'une des revendications 1 à 3, dans lequel une (41) des deux composantes (41, 42) est une matière de base dont une majeure partie de la structure de fibres textile (4) est composée et l'autre (42) des deux composantes (41, 42) est une matière étrangère dont la proportion dans la structure de fibres textile (4) est déterminée.

5. Procédé selon la revendication 4, dans lequel la matière de base (41) est du coton et la matière étrangère (42) est du polyéthylène.

6. Procédé selon l'une des revendications précédentes, dans lequel l'au moins un maximum local se situe à la longueur d'onde ou aux longueurs d'ondes du rayonnement électromagnétique (3) où la valeur absolue de la différence des coefficients d'absorption, des coefficients de transmission ou des coefficients de réflexion des deux composantes (41, 42) comporte un maximum local.

7. Procédé selon l'une des revendications précédentes, dans lequel le coefficient de transmission ou le coefficient de réflexion du filtre spectral (6) comporte au moins deux maxima locaux et deux minima locaux dans la bande du spectre.

8. Procédé selon l'une des revendications précédentes, dans lequel le filtre spectral (6) est conçu comme un filtre à réflexion ou comme un filtre à transmission.

9. Procédé selon l'une des revendications précédentes, dans lequel le filtre spectral (6) est conçu comme un filtre à interférence.

10. Procédé selon l'une des revendications précédentes, dans lequel le filtre spectral (6) est intégré dans le détecteur de rayonnement (8).

11. Procédé selon l'une des revendications précédentes, dans lequel le détecteur de rayonnement (8) a une résolution spatiale et/ou une résolution temporelle.

12. Procédé selon la revendication 11, dans lequel le détecteur de rayonnement (8) est conçu soit comme une caméra numérique munie d'un convertisseur d'images à deux dimensions, soit comme un capteur linéaire à une dimension.
